# EUROPEAN PATENT APPLICATION

(11) **EP 3 957 253 A2**
(43) Date of publication of application: **23.02.2022**
(21) Application number: 21190879.3
(22) Date of filing: 11.08.2021
(51) Int. Cl.: A61B 17/072

(54) **SURGICAL STAPLING DEVICE WITH RELOAD ASSEMBLY HAVING A LOCKOUT**

(30) Priority: 17.08.2020 US 202016994785
(71) Applicant: Covidien LP, Mansfield, MA 02048 (US)
(72) Inventor: BARIL, Jacob C., Norwalk, CT 06851 (US); WHITFIELD, Kenneth H., North Haven, CT 06473 (US); WALCOTT, Zalika, Hamden, CT 06514 (US)
(74) Representative: Maschio, Antonio

(57) **Abstract**

A reload assembly of a surgical stapling device includes a tool assembly, a drive assembly that is movable from a retracted position to an advanced position to actuate the tool assembly, and a lockout assembly including a lock member and a biasing member that prevents readvancement of the drive assembly. The lock member and the biasing member of the lockout assembly are configured to be securely fastened to each other using different structures and methods of securement.

## Description

### FIELD

This disclosure is directed to stapling devices and, more particularly, to a reload assembly having a lockout for use with a stapling device.

### BACKGROUND

Surgical stapling devices for performing surgical procedures endoscopically are well known and are commonly used to reduce patient trauma and shorten patient recovery times. Typically, an endoscopic stapling device includes a handle assembly, an elongate body that extends distally from the handle assembly, and a tool assembly that is supported on a distal portion of the elongate body. In order to reduce costs, the tool assembly is sometimes included as part of a reload assembly that can be removed from the elongate body and replaced to facilitate reuse of the handle assembly and the elongate body.

Typically, reload assemblies include a drive member that is releasably coupled to a control rod included in the elongate body when the reload assembly is coupled to the elongate body. The drive assembly includes a distal portion that is coupled to a clamp member that supports a knife blade and is positioned within the tool assembly such that movement of the drive assembly from a retracted position to an advanced position advances the clamp member through the tool assembly to eject staples from the tool assembly and cut tissue. The reload assembly may include a lockout to prevent readvancement of the drive assembly after the reload assembly has already been fired.

In reload assemblies having lockouts, the lockout may include a lock member that is urged towards the locked position by a biasing member. In some instances, the biasing member includes a flat spring that is welded to the lock member in cantilevered fashion. In cases where the weld does not hold, the lockout may not function properly.

A continuing need exists in the medical arts for a lockout for a reload assembly that includes a biasing member that can be more securely fastened to the lock member.

### SUMMARY

Aspects of the disclosure are directed to a reload assembly including a lockout assembly having a lock member and a biasing member that are securely fastened to each other. Other aspects of this disclosure are directed to methods for securely fastening a biasing member of a lockout assembly to a lock member of the lockout assembly.

One aspect of the disclosure is directed to a reload assembly including a body portion, a tool assembly, a drive assembly, and a lockout assembly. The body portion has a distal body portion and a proximal body portion and defines a channel that extends through the proximal and distal body portions. The tool assembly is supported on the distal body portion and includes an anvil and a cartridge assembly. The drive assembly is supported within the channel of the body portion and includes a drive member and a lockout shield. The drive member defines a stop surface and is movable from a prefired retracted position to an advanced position to actuate the tool assembly, and movable from the advanced position to a post fired retracted position. The lockout shield is positioned about the drive member and is movable from a first position located about the stop surface to a second position exposing the stop surface. The lockout assembly includes a lock member and a biasing member. The lock member is movable from a first position aligned with the stop surface to a second position misaligned with the stop surface. The biasing member is positioned to urge the lock member towards the first position and the lockout shield is positioned to retain the lock member in the second position. The lock member includes a crimp able member to crimp the biasing member to the lock member.

In aspects of the disclosure, the lockout shield includes a lance that is secured to a body of the lockout shield in cantilevered fashion and two resilient arms that engage the drive member to releasably couple the lockout shield to the drive member when the lockout shield is in first position.

In some aspects of the disclosure, the lance extends outwardly from the body of the lockout shield, and movement of the drive member from the prefired retracted position to the post fired retracted position moves the lockout shield from its first position to its second position.

In certain aspects of the disclosure, the proximal body portion is coupled to the distal body portion and includes an abutment member that extends into the channel, and the lance of the lockout shield moves from a position proximal of the abutment member to a position distal of the abutment member when the drive member is moved from the prefired retracted position to the advanced position.

In aspects of the disclosure, the lance engages the abutment member when the drive member is moved from the advanced position towards the post fired retracted position to move the lockout shield from its first position to its second position.

In some aspects of the disclosure, the drive assembly includes a drive block that is secured to the drive member, and the drive block is adapted to releasably engage a control rod of a stapling device.

In certain aspects of the disclosure, the lock member includes a leaf spring.

In aspects of the disclosure, the crimp able member includes a tab and the leaf spring defines an opening, and the tab is received within the opening and crimped onto the leaf spring to secure the leaf spring to the to the lock member.

In some aspects of the disclosure, the tab includes two tabs.

In certain aspects of the disclosure, the lock member defines a through bore and the leaf spring includes flange that defines an opening.

In aspects of the disclosure, the crimp able member includes a rivet that extends through the opening in the flange and through the through bore and is crimped to secure the leaf spring to the lock member.

In some aspects of the disclosure, the leaf spring defines an opening and the crimp able member includes a post supported on the lock member, and the post is received within the opening and crimped to secure the leaf spring to the lock member.

In certain aspects of the disclosure, the post is integrally formed with the lock member.

In aspects of the disclosure, the lock member includes pivot members and is pivotally connected to the proximal body portion.

In some aspects of the disclosure, one end of the lock member defines a concavity and the proximal body portion includes a finger that is received within the concavity.

Another aspect of this disclosure is directed to a lockout assembly that includes a lock member, a leaf spring defining an opening, and a crimp able member that is positioned within the opening of the leaf spring and engaged with the lock member. The crimp able member is crimped to secure the biasing member to the lock member.

Another aspect of this disclosure is directed to a method of fastening a leaf spring to a lock member of a lockout assembly including inserting a crimp able member that extends from the lock member through an opening defined in one end of the leaf spring, and crimping the crimp able member to secure the leaf spring to the lock member.

Other aspects of the disclosure will be appreciated from the following description.

### BRIEF DESCRIPTION OF THE DRAWINGS

Various exemplary aspects of the disclosure are described herein below with reference to the drawings, wherein:
FIG. 1 is a side perspective view of a surgical stapling device including a reload assembly according to aspects of the disclosure;
FIG. 2 is a side perspective view of the reload assembly of the stapling device shown in FIG. 1;
FIG. 3 is side perspective view of a proximal portion of the reload assembly shown in FIG. 2 with a portion of the housing removed, a drive member of the reload assembly in a retracted position and a lockout member of the reload assembly in an unlocked condition;
FIG. 4 is a cross-sectional view taken through the proximal portion of the reload assembly shown in FIG. 3 with the drive member of the reload assembly in an advanced position;
FIG. 5 is a perspective view of the proximal portion of the reload assembly shown in FIG. 3 with the drive member of the reload assembly in a retracted position and the lockout member of the reload assembly in an unlocked condition;
FIG. 6 is a perspective, partial cutaway view of the proximal portion of the reload assembly shown in FIG. 5 with the drive member is a partially advanced position and the lockout member in a locked position engaged with the drive member;
FIG. 7 is a side perspective view of a lockout assembly of the reload assembly shown in FIG. 6 including the lockout member and a biasing member with parts separated;
FIG. 8 is a side perspective view of the lockout assembly shown in FIG. 7 with the biasing member engaged but not secured to the locking member;
FIG. 9 is a side perspective view of the lockout assembly shown in FIG. 8 with the biasing member engaged and secured to the locking member;
FIG. 10 is a side perspective view of another version of the lockout assembly of the reload assembly shown in FIG. 3 with the biasing member secured to the locking member;
FIG. 11 is a side perspective view of yet another version of the lockout assembly of the reload assembly shown in FIG. 3 with the biasing member secured to the locking member;
FIG. 12 is a cross-sectional view taken along section line 12-12 of FIG. 11;
FIG. 13 is a side perspective view of another version of the lockout assembly of the reload assembly shown in FIG. 3 with the biasing member secured to the locking member;
FIG. 14 is a side perspective view of another version of the lockout assembly of the reload assembly shown in FIG. 3 with the biasing member secured to the locking member;
FIG. 15 is a side perspective view of yet another version of the lockout assembly of the reload assembly shown in FIG. 3 with the biasing member secured to the locking member;
FIG. 16 is a side perspective view of another version of the lockout assembly of the reload assembly shown in FIG. 3 with the biasing member secured to the locking member;
FIG. 17 is a side perspective view from one side of another version of the lockout assembly of the reload assembly shown in FIG. 3 with the biasing member secured to the locking member;
FIG. 18 is a side perspective view from the other side of the lockout assembly shown in FIG. 17;
FIG. 19 is a side perspective view of yet another version of the lockout assembly of the reload assembly shown in FIG. 3 with the biasing member secured to the locking member;
FIG. 20 is a side perspective view of the lockout assembly shown in FIG. 19 with the biasing member separated from the locking member;
FIG. 21 is a side perspective view of another version of the lockout assembly of the reload assembly shown in FIG. 3 with the biasing member secured to the lockout member;
FIG. 22 is a side perspective view of the lockout assembly shown in FIG. 21 with the biasing member secured to the locking member;
FIG. 23 is a side perspective view of another version of the lockout assembly of the reload assembly shown in FIG. 3 with the biasing member separated from the locking member; and
FIG. 24 is a side perspective view of the lockout assembly shown in FIG. 26 with the biasing member secured to the locking member.

### DETAILED DESCRIPTION

The disclosed surgical stapling device will now be described in detail with reference to the drawings in which like reference numerals designate identical or corresponding elements in each of the several views. However, it is to be understood that the aspects of the disclosure described herein are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "clinician" is used generally to refer to medical personnel including doctors, nurses, and support personnel.

Aspects of this disclosure are directed to a reload assembly of a surgical stapling device that includes a tool assembly, a drive assembly that is movable from a retracted position to an advanced position to actuate the tool assembly, and a lockout assembly including a lock member and a biasing member that prevents readvancement of the drive assembly. More particularly, aspects of this disclosure are directed to a lockout assembly in which the biasing member is securely fastened to the lock member and to methods for securely fastening the biasing member to the lock member of the lockout assembly.

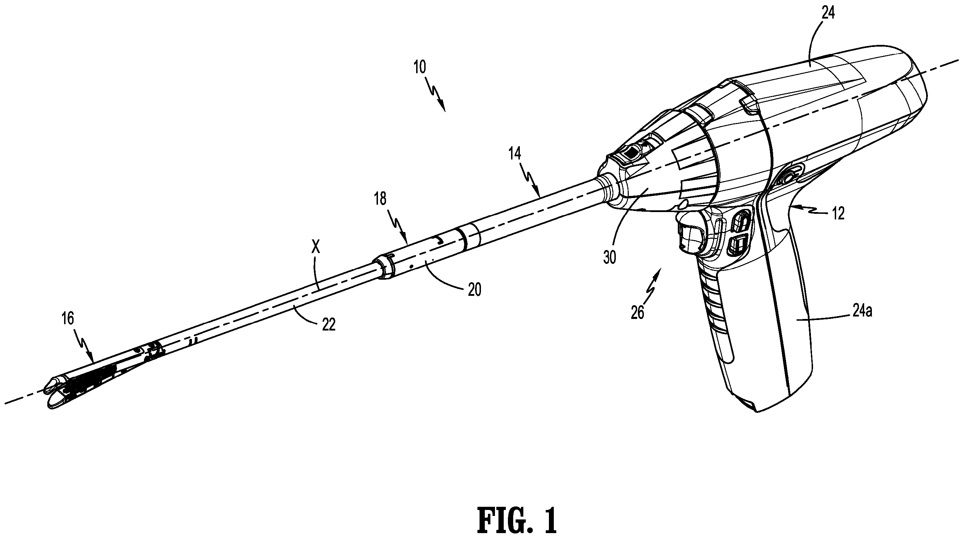

FIG. 1 illustrates a surgical stapling device shown generally as stapling device 10 which includes a handle assembly 12, an elongate body 14 defining a longitudinal axis "X", and a tool assembly 16. The tool assembly 16 forms part of a reload assembly 18 that is releasably coupled to the elongate body 14 and includes a proximal body portion 20, a distal body portion 22, and the tool assembly 16. In aspects of the disclosure, the distal body portion 22 has a diameter that is greater than the diameter of the proximal body portion 20 to facilitate passage of the distal body portion 22 through a small diameter cannula.

The handle assembly 12 includes a body 24 that defines a hand grip 24a and supports actuation buttons 26 that are positioned adjacent the hand grip 24. The actuation buttons 26 are depressible to control operation of the various functions of the stapling device 10 including approximation, firing, and cutting. In aspects of the disclosure, the handle assembly 12 also supports a rotation knob 30 that rotatably couples the elongate body 14 to the handle assembly to facilitate rotation of the elongate body 14 about the longitudinal axis "X" in relation to the handle assembly 12. Although the stapling device 10 is illustrated as an electrically powered stapling device, it is envisioned that aspects of this disclosure are also suitable for use with robotically controlled stapling devices and manually powered stapling devices. U.S. Patent No. 9,055,943 ("the '943 Patent") discloses a surgical stapling device including an electrically powered handle assembly and U.S. Patent No. 6,241,139 ("the '139 Patent") discloses a manually actuated handle assembly.

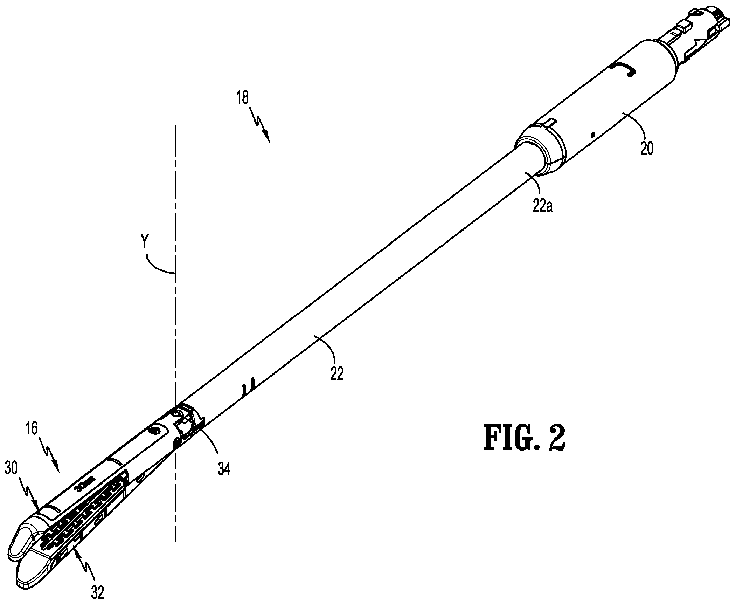

FIG. 2-4 illustrate the reload assembly 18 including the proximal body portion 20, the distal body portion 22, and the tool assembly 16. The tool assembly includes an anvil 30 and a cartridge assembly 32. The cartridge assembly 32 includes a staple cartridge that supports staples (not shown). The anvil 30 and the cartridge assembly 32 are coupled together such that the tool assembly 16 is movable between open and clamped positions. In aspects of the disclosure, the tool assembly 16 is pivotably coupled to a distal end of the distal body portion 22 of the reload assembly 18 about a pivot member 34 that defines an axis "Y" that is substantially perpendicular to the longitudinal axis "X" of the elongate body 14.

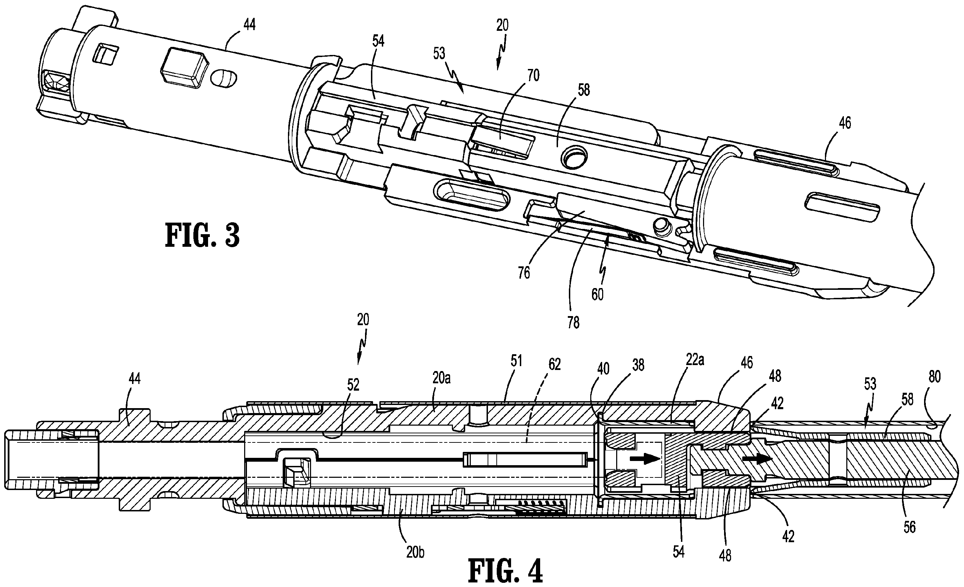

The distal body portion 22 of the reload assembly 18 includes a proximal portion 22a that is coupled to the proximal body portion 20 of the reload assembly 18. In aspects of the disclosure, the proximal portion 22a of the distal body portion 22 includes an annular flange 38 (FIG. 4) that is received within an annular groove 40 (FIG. 4) defined within a distal portion of the proximal body portion 20. The proximal portion 22a of the distal body portion 22 also defines diametrically opposed openings 42 (FIG.4).

FIGS. 3-6 illustrate the proximal body portion 20 of the reload assembly 18 which includes a proximal portion 44 and a distal portion 46. The proximal portion 44 of the proximal body portion 20 of the reload assembly 18 is adapted to releasably engage the distal portion of the elongate body 14. The distal portion 46 of the proximal body portion 20 of the reload assembly 18 includes inwardly extending abutment members 48 (FIG. 4) that extend through the openings 42 (FIG. 4) in the proximal portion 22a of the distal body portion 22 into a channel 50 defined by the distal body portion 22 of the reload assembly 18.

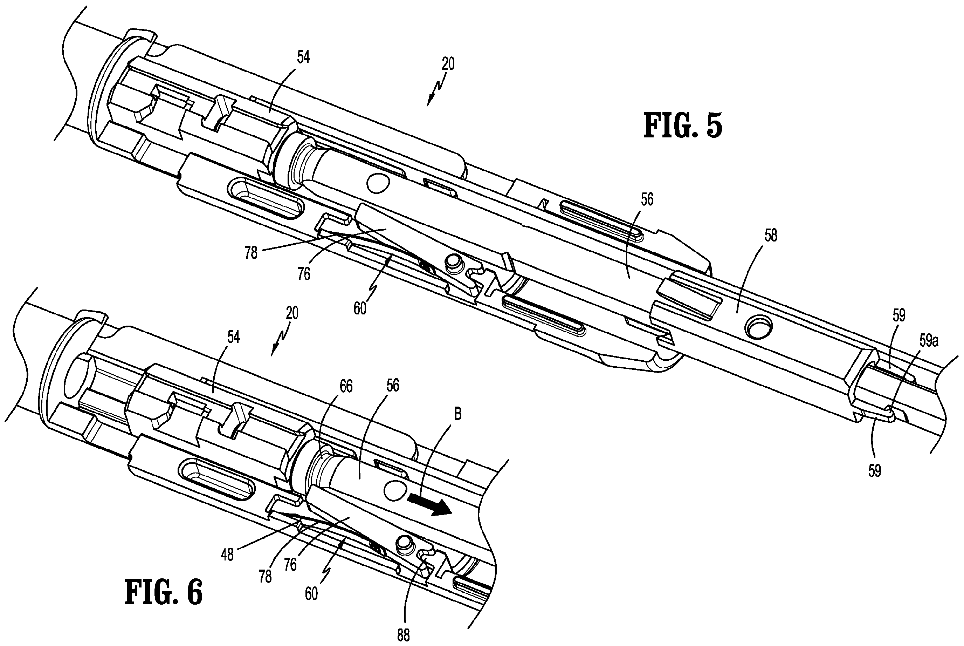

In aspects of the disclosure, the proximal body portion 20 of the reload assembly 18 includes first and second half-sections 20a and 20b (FIG. 4) that are coupled together to define a channel 52. The first and second half sections 20a and 20b are contained within an outer sleeve 51 (FIG. 4). The channel 52 of the proximal body portion 20 receives a drive assembly 53 including a drive block 54, a drive member 56, a lockout shield 58. The proximal body portion 20 also supports a lockout assembly 60. The drive block 54 has a proximal end and a distal end. The proximal end of the drive block 54 is adapted to be releasably coupled to a control rod 62 (FIG. 4) that extends from the elongate body 14 of the stapling device 10 (FIG. 1) such that axial movement of the control rod 62 causes axial movement of the drive block 54. The distal end of the drive block 54 is coupled to the proximal end of the drive member 56 such that axial movement of the drive block 56 causes axial movement of the drive member 56. The drive member 56 includes a proximal portion that forms a stop surface 66 (FIG. 6). In aspects of the disclosure, the stop surface 66 is formed by an annular flange that is formed integrally with or fixedly supported about the drive member 56. It is envisioned the stop surface 66 can be formed in a variety of different manners directly into the drive member 56 or by structure supported on the drive member 56.

The lockout shield 58 is movably positioned about the drive member 56 from a first position covering the stop surface 66 (FIG. 3) of the drive member 56 to a second position spaced longitudinally of the stop surface 66 FIG. 4). The lockout shield 58 includes diametrically opposed lances 70 which are axially aligned with the abutment members 48 formed on the distal portion of the proximal body portion 20. The lances 70 each include a distal end that is secured in cantilevered fashion to a body of the lockout shield 58 such that the lances 70 are deformable inwardly to pass between the abutment members 48 of the proximal body portion 20 when the lockout shield 58 is moved to its advanced position as described below. The lockout shield 58 also includes resilient arms 59 (FIG. 5) that include inwardly extending projections 59a that are received within recesses 56a formed in the drive member 56 to releasably secure the lockout shield 58 to the drive member 56.

The lockout assembly 60 is secured to the proximal body portion 20 of the reload assembly 18 and is movable between a locked position aligned with the stop surface 66 of the drive member 56 to an unlocked position misaligned with the stop surface 66 of the drive member 56. The lockout assembly 60 includes a lock member 76 and a biasing member 78 that is positioned to urge the lock member 76 towards the locked position.

The drive assembly 53 is movable between a retracted position and an advanced position within the channel 52 defined by the proximal body portion 20 and a channel 80 (FIG. 4) defined by the distal body portion 22, in response to actuation of the stapling device 10, to actuate the tool assembly 16, i.e., eject staples and cut tissue. When the reload assembly 18 is in a pre-fired retracted position (FIG. 3), the lockout shield 58 is positioned about the stop surface 66 of the drive member 56 and the lock member 76 is blocked by and retained in its unlocked position by the lockout shield 58. In this position, the lances 70 extend outwardly from the lockout shield 58 and engage a distal face of the drive block 54.

When the stapling device 10 (FIG. 1) is actuated and the control rod 62 (FIG. 4) is advanced distally within the proximal body portion 20, the drive assembly 53 is moved to its advanced position in the direction indicated by arrows "A" in FIG. 4. More specifically, when the control rod 62 is advanced within the channel 52 defined by the proximal body portion 20, the drive block 54 is advanced within the channel 52 of the proximal body portion 20 to advance the drive member 56 within the channel 50 of the proximal body portion 20 and through the channel 80 of the distal body portion 22. As described above, the lockout shield 58 is releasably coupled to the drive member 56 by the resilient arms 59 (FIG. 5) and the lances 70 are engaged with the distal face of the drive block 54. As such distal movement of the drive block 54 also causes distal movement of the lockout shield 58 within the channel 52 of the proximal body portion 20. Movement of the drive member 56 through the distal body portion 22 and through the tool assembly 16 (FIG. 1) actuates the tool assembly 16.

When the lockout shield 58 moves to its advanced position, the lances 70 engage the abutment members 48 and are deformed inwardly and pass distally beyond the abutment members 48. When the lances 70 pass distally beyond the abutment members 48, the lances 70 return to their undeformed conditions in which the lances 70 extend outward of and are aligned with the abutment members 48.

FIG. 5 illustrates the proximal body portion 20 of the reload assembly 20 after the reload assembly has been fired and the drive block 54 and drive member 56 are returned to their retracted positions. When the drive member 56 is returned to its retracted position, the lances 70 of the lockout shield 58 engage the abutment surfaces 48 of the proximal body portion 20 to retain the lockout shield 58 in its advanced position. As such, the stop surface 66 of the drive member 56 is exposed and the lock member 76 is biased to a position engaged with the outer surface of the drive member 56 in axial alignment with the stop surface 66. If the drive member 56 is advanced after the reload assembly 18 has been fired, the lock member 76 engages the stop surface 66 to prevent further distal movement of the drive member 56 in the direction indicated by arrow "B", i.e., the lock member prevents readvancement of the drive member 56. This prevents advancement of a knife blade through the tool assembly 16 (FIG. 1) when staples are not present in the tool assembly 16.

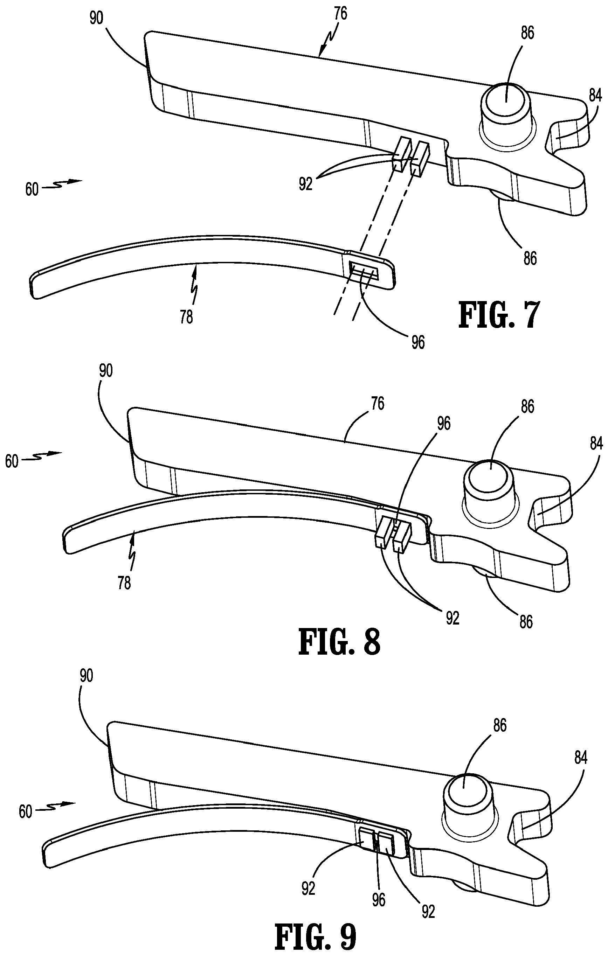

FIGS. 7-10 illustrate the lockout assembly 60 including the lock member 76 and the biasing member 78. In aspects of the disclosure, the lock member 76 includes a body 82 that has a proximal portion and a distal portion. The proximal portion of the body 82 defines a concavity 84 and includes pivot members 86. The pivot members 86 are received between the first and second half sections 20a and 20b, respectively, of the proximal body portion 20 such that the lock member 76 is pivotable about an axis that is substantially perpendicular to a longitudinal axis of the proximal body portion 20. When the pivot members 86 are received between the first and second half-sections 20a and 20b of the proximal body portion 20, the concavity 84 of the lock member 76 receives a finger 88 (FIG. 6) that is formed on the proximal body portion 20 of the reload assembly 18. The finger 88 adds rigidity to the lock member 76 and assists in locating the lock member 76 within the proximal body portion 20. The distal portion of the lock member 76 includes a substantially planar, tapered abutment surface 90. In aspects of the disclosure, the abutment surface 90 is configured such that a plane defined by the abutment surface 90 is substantially perpendicular to the longitudinal axis of the proximal body portion 20 when the lock member 76 is in its locked position.

The body 82 of the lock member 76 includes one or more tabs 92 that extend radially outward from the body 82 when the body 82 is pivotally secured to the proximal body portion 20. In aspects of the disclosure, the biasing member 78 is a leaf spring which stamped from a resilient material such as steel. The leaf spring 78 includes a proximal portion and a distal portion. The distal portion of the leaf spring 78 defines an opening 96 that receives the one or more tabs 92 during manufacturing. After the tabs 92 are received within the opening 96, the tabs 92 are crimped (FIG. 9) to secure the leaf spring 78 to the lock member 76 in cantilevered fashion. When the reload assembly 18 is assembled, the proximal end of the leaf spring 78 engages an inner surface 98 (FIG. 6) of the proximal body portion 20 such that the leaf spring 78 urges the lock member 76 towards the locked position.

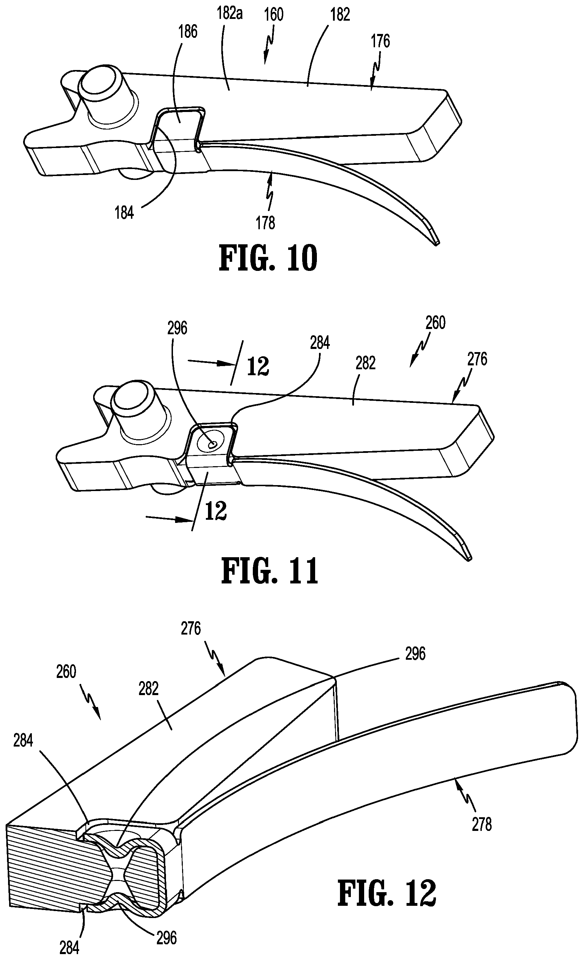

FIG. 10 illustrates an alternate version of the lockout assembly of the reload assembly 20 (FIG. 2) which is shown generally as lockout assembly 160 and includes lock member 176 and a leaf spring 178. The lockout assembly 160 is substantially like the lockout assembly 60 (FIG. 9) except in how the leaf spring 178 is attached to the lock member 176. More specifically, in the lockout assembly 160, the body 182 of the lock member 176 includes a side wall 182a that defines recess 184 and the leaf spring 178 includes a jog or flange 186 that is received within the recess and welded to secure the leaf spring 178 to the lock member 176. The flange 186 and recess 184 significantly increase the weld area for securing the leaf spring 178 to the lock member 176 to more securely secure the leaf spring 178 to the lock member 176.

FIGS. 11 and 12 illustrate another alternate version of the lockout assembly of the reload assembly shown in FIG. 2 which is shown generally as lockout assembly 260. The lock out assembly 260 is substantially like the lockout assembly 160 except that the body 282 of the lock member 276 includes a recess 284 in opposite side walls of the lock member 276 and a through bore 290 that connects the recesses 284 to each other. In addition, the distal end of the leaf spring 278 has spaced flanges 286 that are received within the recesses 284. In order to secure the leaf spring 278 to the locking member 276, the flanges 286 can be punched or deformed to define a dimple 296 that is received in the through bore 290 to secure the flanges 286 within the recesses 284. Alternately, the flanges 286 can deformed to define the dimples 296 and thereafter snap fit onto the locking member 276 within the recesses 284.

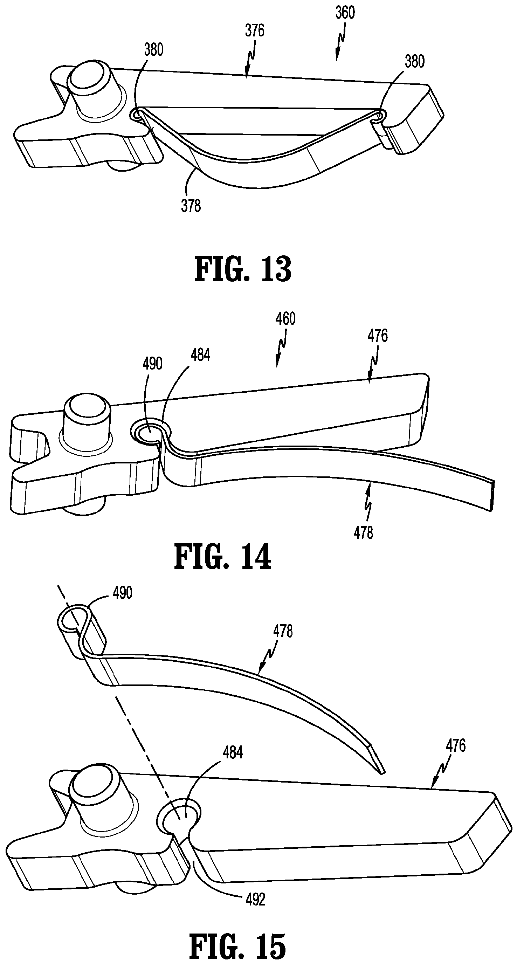

FIG. 13 illustrates another alternate version of the lockout assembly of the reload assembly shown in FIG. 2 which is shown generally as lockout assembly 360. The lockout assembly 360 is substantially like the lockout assemblies described above except that the leaf spring 378 is secured to the lock member 376 at each end of the leaf spring 378. More specifically, each end of the leaf spring 378 includes a coil 380 and the locking member 376 defines spaced pockets 382 that receive the coils 380 of the leaf spring 378 to secure the leaf spring 378 to the lock member 376. The leaf spring 378 has a length that is larger than the distance between the pockets 382 of the lock member 376. As such, when the leaf spring 378 is secured between the pockets 382 of the lock member 376, a central portion 378a of the leaf spring 378 bows outwardly towards the inner surface 98 (FIG. 6) of the proximal body portion 20 of the reload assembly 18.

FIGS. 14 and 15 illustrate another alternate version of the lockout assembly of the reload assembly shown in FIG. 2 which is shown generally as lockout assembly 460. The lockout assembly 460 is substantially like the lockout assemblies described above except that the body 482 of the lock member 476 includes a circular recess 484 that extends transversely across the body 482 of the lock member 476 and the distal end of the leaf spring 478 defines a coil 490 that is received within the circular recess 484. In aspects of the disclosure, the coil 490 can be press fit into the circular recess 484 to securely fasten the leaf spring 476 to the lock member 478. The body 482 of the lock member 476 defines a slot 492 that allows the leaf spring to exit the circular recess 484.

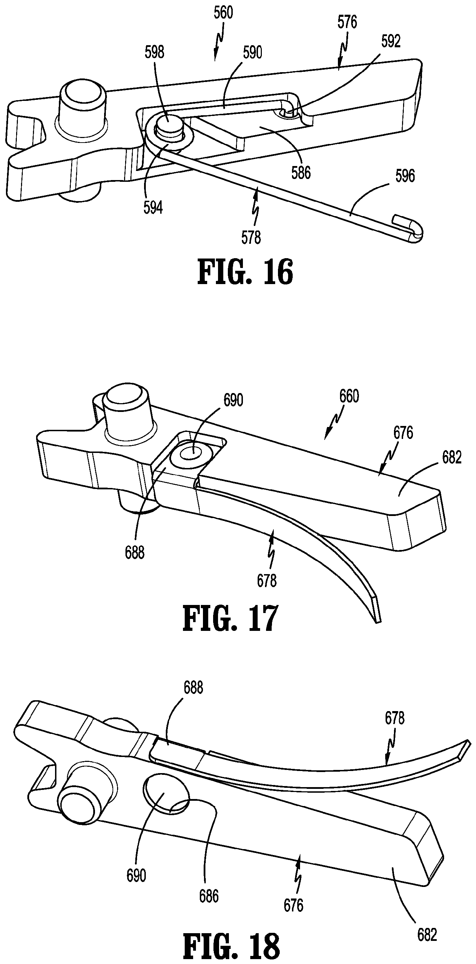

FIG. 16 illustrates another alternate version of the lockout assembly of the reload assembly shown in FIG. 2 which is shown generally as lockout assembly 560. The lockout assembly 560 is substantially like the lockout assemblies described above except that body 582 of the lock member 576 defines a cutout 586 that defines a bore 588 and the biasing member is in the form of a torsion spring 578. The torsion spring 578 includes a first leg 590 that has a transverse bend 592, a central coil portion 594, and a second leg 596 that extends from the cutout 586. The central coil portion 594 of the torsion spring 576 is received about a post 598 that extends outward from the body 582 from within the cutout 586. In aspects of the disclosure, the central coil portion 594 is press fit about the post 598. The bend 592 of the first leg 590 is received in the bore 588 of the body 582 and the first leg 590 is positioned along a wall of the body 582 defining the cutout 586. The second leg 596 of the torsion spring 578 is positioned to engage the inner surface 98 (FIG. 6) of the proximal body portion 20 of the reload assembly 18. In aspects of the disclosure, the second leg 596 includes a bent portion 596a that is spaced from the coil portion 594.

FIGS. 17 and 18 illustrate another alternate version of the lockout assembly of the reload assembly shown in FIG. 2 which is shown generally as lockout assembly 660. The lockout assembly 660 is substantially like the lockout assemblies described above except that body 682 of the lock member 576 defines a recess 684 and a through bore 686, and the leaf spring 678 includes a flange 688 that defines an opening (not shown). The opening receives a rivet 690. When the leaf spring 678 is assembled to the lock member 676, the flange 688 is received within the recess 684 of the body 682 and the rivet 690 is inserted through the opening in the flange 688 of the leaf spring 678 and the through bore 686 of the lock member 676. The rivet 690 can be crimped to secure the leaf spring 578 to the lock member 578.

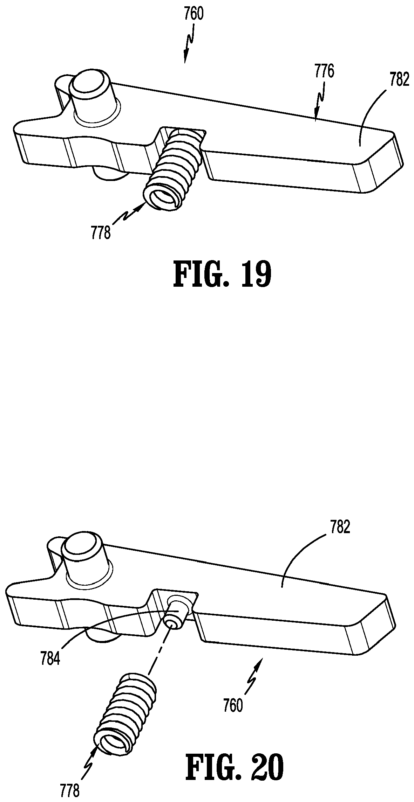

FIGS. 19 and 20 illustrate another alternate version of the lockout assembly of the reload assembly shown in FIG. 2 which is shown generally as lockout assembly 760. The lockout assembly 760 is substantially like the lockout assemblies described above except that the biasing member is in the form of a coil spring 778 and the body 782 of the lock member 776 includes a cutout 782 that includes a post 784. When the coil spring 778 is assembled to the lock member 776, the coil spring 778 is positioned about the post 784 such that the coil spring 778 is compressed between the inner surface 98 (FIG. 6) of the proximal body portion 20 of the reload assembly 18 (FIG. 2) and the lock member 776.

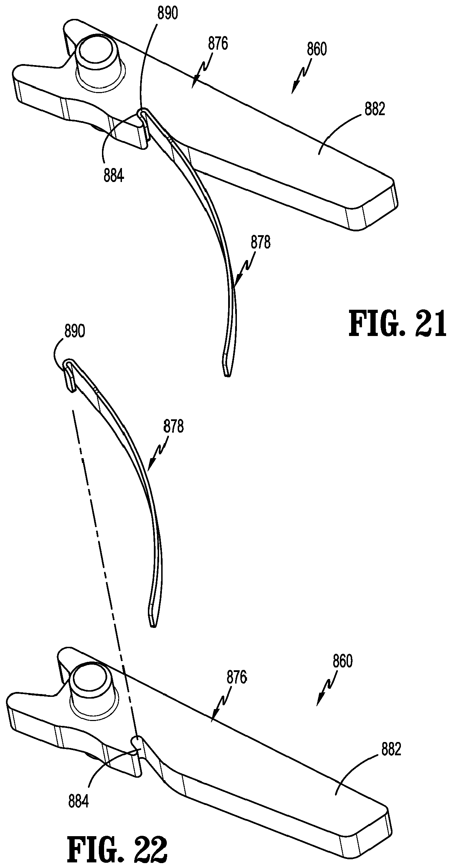

FIGS. 21 and 22 illustrate another alternate version of the lockout assembly of the reload assembly shown in FIG. 2 which is shown generally as lockout assembly 860. The lock out assembly 860 is substantially like the lockout assemblies described above except that the body 882 of the lock member 876 defines an angled slot 884 and the distal end of the leaf spring 888 includes a U-shaped portion 890. The U-shaped portion 890 of the leaf spring 878 is compressed into the angled slot 884 to secure the distal end of the leaf spring 878 to the lock member 876 in cantilevered fashion.

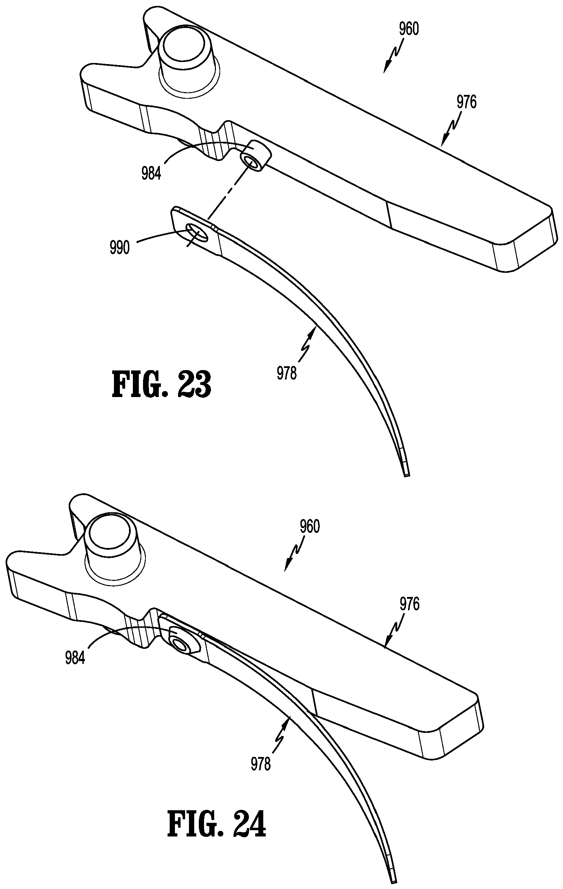

FIGS. 23 and 24 illustrate another alternate version of the lockout assembly of the reload assembly shown in FIG. 2 which is shown generally as lockout assembly 960. The lockout assembly 960 is substantially like the lockout assemblies described above except that the body 982 of the lock member 976 includes a crimp able post 984 and the distal end of the leaf spring 988 defines an opening 990. The crimp able post 984 of the body 982 of the lock member 976 is received within the opening 990 of the leaf spring 978 and crimped (FIG. 24) to secure the leaf spring 978 to the body 982 of the lock member 976.

Persons skilled in the art will understand that the devices and methods specifically described herein and illustrated in the accompanying drawings are non-limiting exemplary aspects of the disclosure. It is envisioned that the elements and features illustrated or described in connection with one exemplary embodiment may be combined with the elements and features of another without departing from the scope of the disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

The invention may be described by reference to the following numbered paragraphs:-
1. A reload assembly comprising:
   a body portion having a distal body portion and a proximal body portion, the body portion defining a channel that extends through the proximal and distal body portions;
   a tool assembly supported on the distal body portion, the tool assembly including an anvil and a cartridge assembly;
   a drive assembly supported within the channel of the body portion, the drive assembly including a drive member and a lockout shield, the drive member defining a stop surface and movable from a prefired retracted position to an advanced position to actuate the tool assembly, and movable from the advanced position to a post fired retracted position, the lockout shield positioned about the drive member and movable from a first position located about the stop surface to a second position exposing the stop surface; and
   a lockout assembly including a lock member and a biasing member, the lock member movable from a first position aligned with the stop surface to a second position misaligned with the stop surface, the biasing member positioned to urge the lock member towards the first position and the lockout shield retaining the lock member in the second position, wherein the lock member includes a crimp able member to crimp the biasing member to the lock member.
2. The reload assembly of paragraph 1, wherein the lockout shield includes a lance that is secured to a body of the lockout shield in cantilevered fashion and two resilient arms, the two resilient arms engaging the drive member to releasably couple the lockout shield to the drive member when the lockout shield is in first position, the lance extending outwardly from the body of the lockout shield, wherein movement of the drive member from the prefired retracted position to the post fired retracted position moves the lockout shield from its first position to its second position.
3. The reload assembly of paragraph 2, wherein the proximal body portion is coupled to the distal body portion and includes an abutment member that extends into the channel, the lance of the lockout shield moving from a position proximal of the abutment member to a position distal of the abutment member when the drive member is moved from the prefired retracted position to the advanced position, the lance engaging the abutment member when the drive member is moved from the advanced position towards the post fired retracted position to move the lockout shield from its first position to its second position.
4. The reload assembly of paragraph 3, wherein the drive assembly includes a drive block secured to the drive member, the drive block adapted to releasably engage a control rod of a stapling device.
5. The reload assembly of paragraph 1, wherein the lock member includes a leaf spring.
6. The reload assembly of paragraph 5, wherein the crimp able member includes a tab and the leaf spring defines an opening, the tab received within the opening and crimped onto the leaf spring to secure the leaf spring to the to the lock member.
7. The reload assembly of paragraph 6, wherein the tab includes two tabs.
8. The reload assembly of paragraph 5, wherein the lock member defines a through bore and the leaf spring includes flange defining an opening, the crimp able member including a rivet that extends through the opening in the flange and through the through bore and is crimped to secure the leaf spring to the lock member.
9. The reload assembly of paragraph 5, wherein the leaf spring defines an opening and the crimp able member includes a post supported on the lock member, the post being received within the opening and crimped to secure the leaf spring to the lock member.
10. The reload assembly of paragraph 9, wherein the post is integrally formed with the lock member.
11. The reload assembly of paragraph 1, wherein the lock member includes pivot members and is pivotally connected to the proximal body portion.
12. The reload assembly of paragraph 11, wherein one end of the lock member defines a concavity and the proximal body portion includes a finger that is received within the concavity.
13. A lockout assembly comprising:
   a lock member;
   a leaf spring defining an opening; and
   a crimp able member positioned within the opening of the leaf spring and engaged with the lock member, the crimp able member being crimped to secure the biasing member to the lock member.
14. The lockout assembly of paragraph 13, wherein the crimp able member includes a tab supported on the lock member, the tab received within the opening and crimped onto the leaf spring to secure the leaf spring to the to the lock member.
15. The lockout assembly of paragraph 14, wherein the tab includes first and second tabs.
16. The lockout assembly of paragraph 13, wherein the lock member defines a through bore and the leaf spring includes flange that defines the opening, the crimp able member including a rivet that extends through the opening in the flange and through the through bore and is crimped to secure the leaf spring to the lock member.
17. The lockout assembly of paragraph 13, wherein the crimp able member includes a post supported on the lock member, the post being received within the opening and crimped to secure the leaf spring to the lock member.
18. The lockout assembly of paragraph 17, wherein the post is integrally formed with the lock member.
19. The lockout assembly of paragraph 13, wherein the lock member is pivotally connected to the proximal body portion, the lock member defining a concavity and the proximal body portion including a finger that is received within the concavity.
20. A method of fastening a leaf spring to a lock member of a lockout assembly, the method comprising:
   inserting a crimp able member that extends from the lock member through an opening defined in one end of the leaf spring; and
   crimping the crimp able member to secure the leaf spring to the lock member.

## Claims

1. A reload assembly comprising:
a body portion having a distal body portion and a proximal body portion, the body portion defining a channel that extends through the proximal and distal body portions;
a tool assembly supported on the distal body portion, the tool assembly including an anvil and a cartridge assembly;
a drive assembly supported within the channel of the body portion, the drive assembly including a drive member and a lockout shield, the drive member defining a stop surface and movable from a prefired retracted position to an advanced position to actuate the tool assembly, and movable from the advanced position to a post fired retracted position, the lockout shield positioned about the drive member and movable from a first position located about the stop surface to a second position exposing the stop surface; and
a lockout assembly including a lock member and a biasing member, the lock member movable from a first position aligned with the stop surface to a second position misaligned with the stop surface, the biasing member positioned to urge the lock member towards the first position and the lockout shield retaining the lock member in the second position, wherein the lock member includes a crimp able member to crimp the biasing member to the lock member.

2. The reload assembly of claim 1, wherein the lockout shield includes a lance that is secured to a body of the lockout shield in cantilevered fashion and two resilient arms, the two resilient arms engaging the drive member to releasably couple the lockout shield to the drive member when the lockout shield is in first position, the lance extending outwardly from the body of the lockout shield, wherein movement of the drive member from the prefired retracted position to the post fired retracted position moves the lockout shield from its first position to its second position.

3. The reload assembly of claim 2, wherein the proximal body portion is coupled to the distal body portion and includes an abutment member that extends into the channel, the lance of the lockout shield moving from a position proximal of the abutment member to a position distal of the abutment member when the drive member is moved from the prefired retracted position to the advanced position, the lance engaging the abutment member when the drive member is moved from the advanced position towards the post fired retracted position to move the lockout shield from its first position to its second position.

4. The reload assembly of claim 3, wherein the drive assembly includes a drive block secured to the drive member, the drive block adapted to releasably engage a control rod of a stapling device.

5. The reload assembly of any preceding claim, wherein the lock member includes a leaf spring; preferably wherein the crimp able member includes a tab and the leaf spring defines an opening, the tab received within the opening and crimped onto the leaf spring to secure the leaf spring to the to the lock member.

6. The reload assembly of claim 5, wherein the tab includes two tabs; and/or wherein the lock member defines a through bore and the leaf spring includes flange defining an opening, the crimp able member including a rivet that extends through the opening in the flange and through the through bore and is crimped to secure the leaf spring to the lock member.

7. The reload assembly of claim 5 or claim 6, wherein the leaf spring defines an opening and the crimp able member includes a post supported on the lock member, the post being received within the opening and crimped to secure the leaf spring to the lock member; preferably wherein the post is integrally formed with the lock member.

8. The reload assembly of any preceding claim, wherein the lock member includes pivot members and is pivotally connected to the proximal body portion.

9. The reload assembly of claim 8, wherein one end of the lock member defines a concavity and the proximal body portion includes a finger that is received within the concavity.

10. A lockout assembly comprising:
a lock member;
a leaf spring defining an opening; and
a crimp able member positioned within the opening of the leaf spring and engaged with the lock member, the crimp able member being crimped to secure the biasing member to the lock member.

11. The lockout assembly of claim 10, wherein the crimp able member includes a tab supported on the lock member, the tab received within the opening and crimped onto the leaf spring to secure the leaf spring to the to the lock member; preferably wherein the tab includes first and second tabs.

12. The lockout assembly of claim 10 or claim 11, wherein the lock member defines a through bore and the leaf spring includes flange that defines the opening, the crimp able member including a rivet that extends through the opening in the flange and through the through bore and is crimped to secure the leaf spring to the lock member.

13. The lockout assembly of any of claims 10 to 12, wherein the crimp able member includes a post supported on the lock member, the post being received within the opening and crimped to secure the leaf spring to the lock member; preferably wherein the post is integrally formed with the lock member.

14. The lockout assembly of any of claims 10 to 13, wherein the lock member is pivotally connected to the proximal body portion, the lock member defining a concavity and the proximal body portion including a finger that is received within the concavity.

15. A method of fastening a leaf spring to a lock member of a lockout assembly, the method comprising:
inserting a crimp able member that extends from the lock member through an opening defined in one end of the leaf spring; and
crimping the crimp able member to secure the leaf spring to the lock member.
